# EUROPÄISCHE PATENTSCHRIFT

(11) **EP 3 463 178 B1**
(45) Veröffentlichungstag und Bekanntmachung des Hinweises auf die Patenterteilung: **26.08.2020**
(21) Anmeldenummer: 17725262.4
(22) Anmeldetag: 23.05.2017
(51) Int. Cl.: A61C 13/083, A61C 5/20

(54) **VERFAHREN ZUR HERSTELLUNG VON ZAHNERSATZ**
METHOD FOR PRODUCING DENTURES
PROCÉDÉ DE FABRICATION DE PROTHÈSE DENTAIRE

(30) Priorität: 23.05.2016 DE 102016109447; 23.05.2016 DE 102016109449
(43) Veröffentlichungstag der Anmeldung: 10.04.2019
(73) Patentinhaber: Bredent GmbH & Co. KG, 89250 Senden (DE)
(72) Erfinder: MEYER, Joachim, 87751 Heimertingen (DE)
(74) Vertreter: Baur & Weber Patentanwälte PartG mbB
(86) Internationale Anmeldenummer: PCT/EP2017/062469
(87) Internationale Veröffentlichungsnummer: WO 2017/202869

(56) Entgegenhaltungen:
- EP-A2- 0 998 882
- DE-A1- 3 524 783
- US-A- 5 346 397
- US-A1- 2004 245 663
- US-A1- 2009 035 726

## Beschreibung

Die Erfindung betrifft ein Verfahren zur Herstellung von Zahnersatz, insbesondere mittels eines keramischen Halbzeugs oder mittels einer anatomisch geformten Keramikschale als Verblendung eines Dentalgerüstes oder als vollkeramischer Zahnersatz.

Die Verwendung von keramischen Materialien ist in der Zahntechnik weit verbreitet. Neben der Verwendung in der Prothetik, wie z. B. für Einzelkronen, werden beispielsweise auch Verblendschalen (sog. Veneers) aus Keramik hergestellt.

Aus der WO 2007/028 787 A1 ist keramischer Zahnersatz und ein Verfahren zu dessen Herstellung bekannt. In dieser Druckschrift wird Zahnersatz, insbesondere in Form von einer Verbundkrone oder einer Verbundbrücke beschrieben, der aus zwei unabhängigen Bestandteilen besteht, die als innere Gerüststruktur und äußere Verblendhülle ausgebildet sind, welche durch eine Konnektormasse miteinander verbunden sind. Die Konnektormasse ist dabei bei Zimmertemperatur flüssig oder zumindest zähflüssig, um die Verarbeitung zu vereinfachen.

Aus der DE 600 23 315 T2 ist ein Verfahren zur Herstellung eines funktionalen Dentalelements bekannt, bei dem Schichten aus einem geeigneten keramischen Material, das ein Pulver ist, nacheinander aufgebracht werden. Dabei wird mittels einer dreidimensionalen Drucktechnik ein Binder auf jede Schicht aus Pulver an gewünschten Positionen aufgebracht, um jede Schicht aus Pulver an die vorhergehende Schicht zu binden, wodurch die Entfernung von überschüssigen, nicht-haftendem Pulver erlaubt wird. Anschließend wird das so gebildete Dentalelement einem Sinterschritt unterzogen und das gesinterte Element wird durch eine zweite Phase infiltriert. Auf den Sinterschritt kann auch ein Entbindungsschritt folgen.

Aus der DE 10 2009 051 593 A1 ist ein Haftvermittler zwischen einer Oxidkeramik und einem Verblendwerkstoff, insbesondere für dentale Zwecke, bekannt. Dabei wird auf einen zu verblendenden und noch nicht dichtgesinterten Grundkörper aus Oxidkeramik oder deren Ausgangsmaterialien ein Haftvermittler in Form eines Gemisches aus Silikatkeramik und Quarz als Sol aufgetragen. Anschließend wird der Grundkörper mit dem eingewirkten Haftvermittler endgesintert und danach der Verblendwerkstoff aufgebracht. Dadurch können hoch belastbare dentale Kronen oder Brücken hergestellt werden.

In der US 2004/0245663 A1 wird ein grünkeramisches Band beschrieben, welches zur Herstellung von Zahnersatz verwendet werden kann. Das Band wird zu diesem Zweck um ein Modell eines Zahnstumpfes gelegt und anschließend angepasst und gebrannt.

Aus der US 5 346 397 A ist ein Verfahren zur Herstellung von Zahnersatz bekannt, bei dem in eine noch nicht angepasste sowie ungebrannte Keramikschale zusammen mit einer keramischen Paste als Aufbaumaterial verwendet wird, sodass ein farblich und formpassender aber noch ungebrannter Zahnersatz entsteht.

In der EP 0 826 642 A2 wird die Herstellung einer keramischen Zahnersatzkrone oder einer keramischen Zahnersatzschale beschrieben, bei der ein Schlicker zu einer dünnen Lage geformt wird, diese dünne Lage auf eine Gipsform gegeben, Lage für Lage getrocknet und nach Auftragung aller erforderlichen Lagen der Rohling gesintert wird.

In der DE 3524783 wird eine keramische Zusammensetzung zur Herstellung von Zahnersatz beschrieben, aus der eine Zahnfacette gefertigt werden kann. Diese ist durch ihre plastische Konsistenz noch am Gerüst veränderbar.

Die EP 0998882 beschreibt ein Verfahren zur Herstellung eines Zahnimplantats, bei welchem ein Keramik-Formteil über eine keramische Masse auf ein Gerüst oder ein Zahnstumpf-Modell aufgebracht wird. Das Keramik-Formteil wird dabei als Prefacette oder Keramikschale auf der Labial- oder Buccalseite aufgebracht und von der keramischen Masse umhüllt. Dieses Zwischenprodukt wird dann mit einem Vorwall aus Silicon positioniert, und die Prefacette wird zusammen mit der Keramikmasse zu einer fertigen Krone ausgearbeitet. Der Vorwall wird entfernt und die Krone wird gebrannt.

Die US 2009/035726 betrifft ein Verfahren zur Herstellung einer Keramikkappe, bei dem ein Zahnmodell mit mindestens einer Kunststoffschicht bedeckt und diese beschichtet wird, um eine Keramikkappe im grünen Zustand zu bilden, welche anschließend gesintert, beschichtet und gebrannt wird.

US 5,346,397 zeigt ein Verfahren zur Herstellung von künstlichen Porzellanzähnen oder laborgefertigten Zahnkronen und beinhaltet das Formen von zahnförmigen ungebrannten Schalen zu Formen vorbestimmter Zahnkonturen, mit einem Füllmedium zu Zahnformen verschmolzen werden. Das Verfahren beinhaltet vorzugsweise eine Anpassung der Zahnfarbe durch das Füllmedium.

Halbzeuge aus keramischen Materialien sind in der Dentaltechnik weit verbreitet und werden bisher üblicherweise mittels CAD/CAM-Bearbeitungen aus dichtgesintertem Material eingesetzt, um vollkeramische Zahnersatzprodukte herstellen zu können.

Aus der DE 10 2006 034 551 A1 ist ein Keramik-Weißling zur Herstellung eines Keramik-Formteils und ein Verfahren zur Herstellung eines Keramik-Weißlings bekannt, wobei durch Vorsintern eines zu einem Grünling gepressten Keramik-Rohmaterials der Keramik-Weißling gebildet wird, der nach dem Vorsintern zu dessen Verstärkung mit einem Zusatzmaterial verstärkt wird, welches bei einer späteren Behandlung bzw. Bearbeitung des Weißlings rückstandsfrei wieder entfernbar ist. Das Zusatzmaterial wird in einem fließfähigen Zustand in Poren des Weißlings eingebracht und anschließend ausgehärtet. Dabei kann es sich um ein Polymer oder ein Kunstharz handeln.

Aus der Technik bekannte Halbzeuge werden als Weißling oder dichtgesintert angeboten. Ein Weißling führt aufgrund seiner sehr spröden Struktur leicht zu Abplatzungen und Rissen, was die spanende Verarbeitung schwierig und zeitaufwendig gestaltet. Dichtgesinterte Halbzeuge benötigen zudem spezielle Verarbeitungssysteme mit Kühlung und zusätzlichem Schleifen. Demnach sind derartige Arbeiten aufgrund spezieller Verarbeitungssysteme in der Verarbeitung und die Gefahr von Beschädigungen durch Risse oder Abplatzungen sind hoch. Des Weiteren sind die Bearbeitungszeiten sehr lang.

Es ist Aufgabe der Erfindung, ein Verfahren zur Herstellung von Zahnersatz anzugeben, bei dem eine einfachere Verarbeitung möglich ist.

Diese Aufgabe wird durch den unabhängigen Patentanspruch 1 gelöst. Weitere vorteilhafte Ausgestaltungen der Erfindung sind jeweils Gegenstand der Unteransprüche. Diese können in technologisch sinnvoller Weise miteinander kombiniert werden. Die Beschreibung, insbesondere im Zusammenhang mit der Zeichnung, charakterisiert und spezifiziert die Erfindung zusätzlich.

Gemäß der Erfindung wird ein Verfahren zur Herstellung von Zahnersatz, insbesondere mittels eines keramischen Halbzeugs oder einer anatomisch geformten Keramikschale als Verblendung eines Dentalgerüstes oder als vollkeramischer Zahnersatz, angegeben, das die folgenden Schritte umfasst: Bereitstellung einer adaptierfähigen anatomisch geformten Keramikschale mit einer Kontaktfläche und einer gegenüberliegenden Außenfläche einer vestibulären, anatomischen Zahnform als plastisch formbaren Grünling; Aufbringen der Keramikschale an ein Gerüst oder an einen Träger mittels einer viskosen keramischen Füllmasse als Ausgleichsmasse, wobei die Keramikschale mit der viskosen keramischen Füllmasse hinterspritzt und auf das Gerüst oder den Träger gesetzt wird; und Durchführen wenigstens eines Brennschritts unterhalb einer Temperatur von 1050°C.

Das erfindungsgemäße Verfahren zur Herstellung von Zahnersatz verwendet einen Grünling, der formbar ist und daher als flexibles Verblendmaterial der Gerüstkontur plastisch angepasst werden kann.

Gemäß einer Ausführungsform der Erfindung wird zusätzlich ein Adaptieren der anatomisch geformten Keramikschale an das Gerüst oder des Träger durchgeführt.

Gemäß einer weiteren Ausführungsform der Erfindung weist die keramische Füllmasse eine farbgebende Eigenschaft auf.

Gemäß einer weiteren Ausführungsform der Erfindung umfasst der Schritt des Bereitstellens einer anatomisch geformten Keramikschale eine spanende Bearbeitung aus einem Halbzeug, das als Grünling aus einer Mischung umfassend ein Keramikpulver und ein Bindersystem gefertigt ist.

Gemäß einer weiteren Ausführungsform der Erfindung umfasst der Schritt des Bereitstellens einer anatomisch geformten Keramikschale ein Bereitstellen eines Keramikschlickers, ein Aufbereiten des Keramikschlickers zu einem Schlickerfilm, ein eventuelles Vortrocknen des Schlickerfilms zur Anpassung des Feuchtigkeitsgrades oder der Viskosität, ein Bilden einer konkaven flexiblen Keramikschale in anatomischer Form und eine Entnahme der flexiblen Keramikschale in Form eines Grünlings.

Gemäß einer weiteren Ausführungsform der Erfindung erfolgt zum Bilden der konkaven Keramikschale ein Eindrücken des Schlickerfilms in eine Negativ-Form, ein Tiefzeihen oder ein Einbringen in Formplatten.

Gemäß einer weiteren Ausführungsform der Erfindung erfolgt das Bilden der konkaven Keramikschale mittels eines 3D-Druckverfahrens.

Gemäß einer weiteren Ausführungsform der Erfindung erfolgt der Schritt des Bereitstellens einer anatomisch geformten Keramikschale mit wenigstens zwei unterschiedlich gefärbter Schichten.

Gemäß einer weiteren Ausführungsform der Erfindung ist der Grünling oder ein aus dem Grünling hervorgegangener Weißling infiltriert.

Gemäß einer weiteren Ausführungsform der Erfindung erfolgt die Infiltration mit einer Glas- oder Keramik-Suspension vor dem Brennschritt.

Gemäß einer weiteren Ausführungsform der Erfindung erfolgt die Infiltration mittels eines Polymers oder Harz nach dem Brennschritt.

Gemäß einer weiteren Ausführungsform der Erfindung wird ein Brandschritt zum Dichtsintern durchgeführt.

Eine Verwendung von keramischem Halbzeug und spanender Bearbeitung oder von einer Keramikschale mit bereits fertiggestellter äußerer Form ist bezüglich Dimensionsstabilität oder Passgenauigkeit gut für die Herstellung von Zahnersatz geeignet, wobei andere Verfahren, wie beispielsweise additiven Produktionsformen keramischen Restaurationen ebenso möglich sind. Insbesondere mit einer dem Wärmeausdehnungskoeffizienten und dem Schrumpfungsverhalten angepasster viskosen keramischen Füllmasse, können mögliche Konstruktionsfehler innerhalb der CAD, oder Bearbeitungsfehler der Zerspanung vor dem Dichtsintern ausgeglichen und damit korrigiert werden.

Dabei wird in der Anwendung der Grünling oder der daraus hervorgehende Weißling vor dem Dichtsintern mit der viskosen Füllmasse hinterspritzt auf das dentale Gerüst oder dem Träger (z.B. feuerfeste Masse) lagerichtig zurückgesetzt. Die viskose Füllmasse (Keramik/Glas-Sol) soll hier ungleiche Spaltmaße beziehungsweise Hinterschnitte des Gerüstes oder des als feuerfeste Masse bereitgestellten Formenkörpers ausgleichen, um die dadurch hervorgerufenen Einfallstellen an der Verblendung zu vermeiden.

Aufgrund seiner plastischen Verformbarkeit ergeben sich deutlich kürzere Verarbeitungszeiten und geringe Neigungen zu Rissen oder Brüchen. Die Eigenschaft der plastischen Verformbarkeit ergibt wesentliche Vorteile in einer nachträglichen händischen korrektiven Formanpassung an den Träger oder Gerüst. Aufgrund der hier möglichen Grünbearbeitung ergeben sich kürzere Bearbeitungszeiten der Zerspanung, da der Grünling nicht die Sprödigkeit von Weißlingen oder dichtgesinterten Halbzeugen aufweist und daher mit schnelleren Verarbeitungsparametern der Zerspanung (Drehzahl/Vorschub/Zustellung) bearbeitet werden kann. Der Grünling kann trocken auf allen Maschinensystemen verarbeitet werden. Demnach wird ein keramisches Halbzeug zur Bildung einer gespanten keramischen Restauration verwendet, wobei nachträglich nach dem Spanprozess eine formkorrigierende Anpassung an den Träger, beispielsweise ein Dentalgerüst oder einen feuerfesten Formkörper, möglich ist. Demnach ist ein Anadaptieren, wie es in der Zahntechnik oftmals vorgenommen werden muss, ohne weiteres möglich, da die Keramikschale nach dem Spanprozess noch verformbar ist und gebogen werden kann. Dies wird durch den Binderzusatz des Grünlings ermöglicht. Das Halbzeug kann als eine Fräsblankform, beispielsweise mit 100 mm Durchmesser und 14 mm Dicke bereitgestellt werden. Dabei wird ein Keramikschlicker mit einem Bindersystem verwendet, wobei der Keramikschlicker aus dentalüblichem Keramikmaterial entsprechend pastös aufbereitet ist. Die spanende Bearbeitung eines Grünlings ist ohne großen Aufwand möglich und stellt keine besonderen Anforderungen hinsichtlich des zu verwendeten Werkzeugs. Demnach ist eine einfache aber sichere Verarbeitung möglich, die schnellere Bearbeitungszeiten bei gleichzeitig geringerer Wahrscheinlichkeit von Brüchen, Abplatzungen oder Rissen im Spanprozess aufweist.

Der Grünling kann monochrom sein, so dass sich Zahnersatz bilden lässt, der mit einer einzigen Farbgebung versehen ist. Mehrere Schichten des Grünlings, können aber auch unterschiedliche Farben aufweisen.

Demnach ist es möglich, die Farbgebung des keramischen Halbzeugs entsprechend anzupassen, um beispielsweise Farbunterschiede zwischen dem Schmelz- und Dentinbereich einer Verblendschale entsprechend anpassen zu können. Die unterschiedlichen Schichten des Grünlings werden dabei nacheinander beispielsweise gegossen, wobei zwischen dem Aufbringen der nachfolgenden Schicht ein Antrocknen der bereits vorhandenen Schlickerschicht erfolgt, um eine separat darüber liegende Schicht gießen zu können. Gegebenenfalls ist ein nachfolgender Pressvorgang oder eine Druckbehandlung durch nachträgliches Nachverdichten mittels Druckkammer oder Überdruck notwendig, um die Grenzflächen der einzelnen Schichten besser zu verbinden und einen geringeren Schrumpf zu erreichen. Dabei könnte insbesondere ein uniaxiales, biaxiales oder isostatisches Pressverfahren zum Einsatz kommen.

Gemäß einer weiteren Ausführungsform der Erfindung ist der Grünling oder ein aus dem Grünling entstandener Weißling infiltriert. Die Infiltration kann dabei vor oder nach dem Brennschritt mit einer Glas- oder Keramik-Suspension sowie mittels eines Polymers oder Harz erfolgen.

Hierbei ist ein weiterer wichtiger Punkt in der Entwicklung von keramischem Zahnersatz darin zu sehen, dass die grundsätzliche Möglichkeit einer Infiltration mittels Glas, Keramik oder Harz besteht, die vor oder nach einem Brennschritt durchgeführt werden kann. Dies ist gegenwärtig mit handelsüblichen Keramiken nicht möglich, da diese in Pulverform mit einer Wasserbasis aufgemischt werden. Durch die Wassersättigung bzw. Löslichkeit einer unmittelbaren Infiltration, ist dies nicht möglich. Auch sind Porositäten und Homogenitäten so ungleichmäßig, dass beispielsweise eine gleichmäßige Farbgebung nicht zu erzielen wäre. Eine Infiltration beseitigt bzw. minimiert mögliche Porosität, wodurch die mechanische Stabilität der Keramik deutlich gesteigert wird. Falls die Infiltration mittels Glas-/Keramik-Suspension umgesetzt wird, ist ein nachfolgendes Brennen erforderlich. Die Infiltration kann auch mittels eines Polymers oder Harz nach dem Brennen der Keramik erfolgen, wobei ein weiteres Brennen nicht mehr möglich ist.

Es ist erfindungsgemäß ebenso vorgesehen, eine chromatische Farbgebung des Keramik-Sols, also zahnfarbigen, chromatischen Glas-Sols zur farblichen Anpassbarkeit der Verblendschale zu schaffen. Eine Besonderheit hier ist, dass die Farbwirkung vom Gerüst oder Träger ausgeht, was der anatomischen Farbgebung entspricht. Im Gegensatz dazu wird im Stand der Technik zur Farbanpassung nach dem Brennen oberflächlich auf die anatomische Außenfläche eine Farbschicht aufgemalt.

Nachfolgend werden einige Ausführungsbeispiele anhand der Zeichnung näher erläutert. Es zeigen:
- Fig. 1: ein keramisches Halbzeug gemäß einer ersten Ausführungsform der Erfindung in einer perspektivischen Seitenansicht,
- Fig. 2: ein keramisches Halbzeug gemäß einer zweiten Ausführungsform der Erfindung in einer perspektivischen Seitenansicht,
- Fig. 3: eine aus dem Halbzeug hergestellte Keramikschale zusammen mit einem Verblendgerüst, und
- Fig. 4: eine aus dem Halbzeug hergestellte Keramikschale zusammen mit einem Formkörper.

In den Figuren sind gleiche oder funktional gleich wirkende Bauteile mit den gleichen Bezugszeichen versehen.

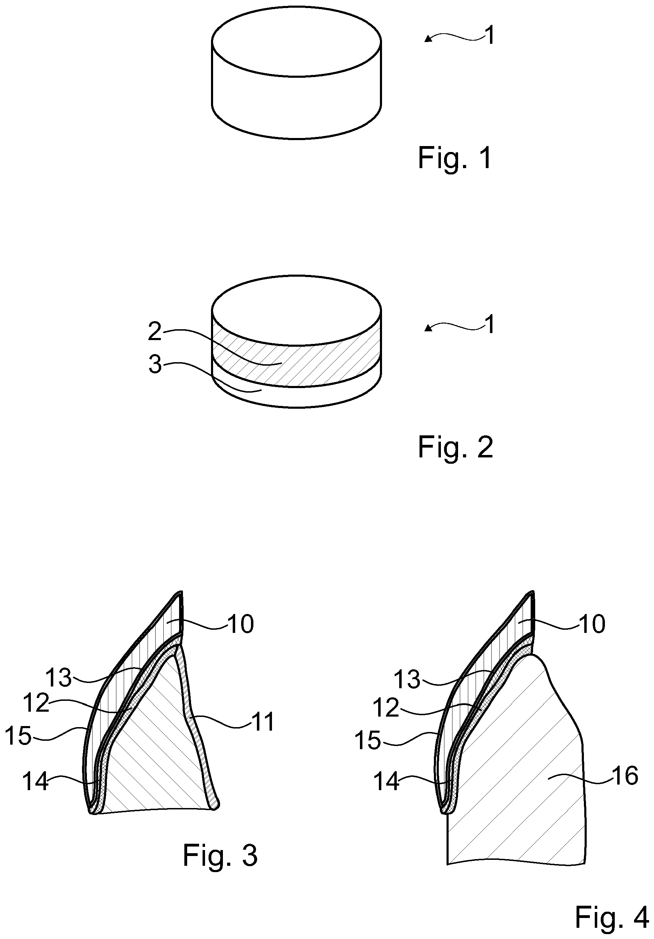

Unter Bezugnahme auf Fig. 1 ist ein keramisches Halbzeug 1 in einer perspektivischen Seitenansicht gezeigt. Das keramische Halbzeug 1 ist als Keramikschlicker aus Keramikpulver mit einem Bindersystem gegossen, das mittels spanender Bearbeitung aus dem so entstandenen Grünling in eine adaptierbare Keramikschale umarbeitbar ist, die vor einem Brennschritt unterhalb einer Temperatur von 1050°C an einen Träger formbar und plastisch anpassbar ist.

Unter Bezugnahme auf Fig. 2 wird eine zweite Ausführungsform gezeigt. Hier werden Fräsblankformen als keramisches Halbzeug 1 bereitgestellt, bei denen separat nacheinander polychrome Schichten 2 gebildet werden, wobei nach jedem Gießvorgang die Keramikschlickerschicht angetrocknet wird, um eine separate darüber liegende Schicht 2 zu gießen. In einer Variante des Verfahrens kann ein nachfolgender feuchter Pressvorgang bzw. ein Verdichten durch Druckluft oder Vibration durchgeführt werden, um die Grenzflächen der Schichten 2 besser zu verbinden und hier einen geringeren Schrumpf zu erreichen.

Somit ist es möglich, sowohl aus dem monochromen Blank gemäß Fig. 1 als auch aus dem polychromen Blank gemäß Fig. 2 individuelle Verblendschalen mittels spanender Bearbeitung zu formen und an einem Verblendgerüst zu fixieren oder an ein feuerfestes Formgerüst anzubringen. Mittels einer farblich unterschiedlichen keramischen Füllmasse kann hierbei eine zusätzliche Färbung geschaffen werden. Eine Verbindung zwischen den Verblendschalen und dem Gerüst bzw. dem Formkörper erfolgt dabei in einem Brennvorgang. Dabei ist jedoch eine plastische formkorrigierende Anpassung möglich, da die Verblendschale nach dem Spanprozess noch verformbar ist.

Anstelle von keramischem Halbzeug 1 kann aber auch eine Keramikschale bereitgestellt werden, die in anatomisch korrekter Form gebildet wurde. Hierzu wird auf die bisher unveröffentlichte Anmeldung DE 10 2016 109 447.1 der gleichen Anmelderin verwiesen. Eine Herstellung einer Keramikschale in einem additiven Verfahren, beispielsweise in einem 3D-Druckverfahren wäre ebenfalls möglich.

Die Verwendung einer flexiblen Keramikschale 10, die wie oben beschrieben hergstellt sein kann, wird nachfolgend unter Bezugnahme auf Fig. 3 näher erläutert.

In Fig. 3 ist schematisch ein Verblendgerüst 11 gezeigt, das eine entsprechende konvexe Verblendfläche 12 aufweist. Auf diese konvexe Verblendfläche 12 wird die Keramikschale 10 aufgesetzt. Die Keramikschale weist eine konkave Kontaktfläche 13 auf, wobei zwischen der konkaven Kontaktfläche 13 und der konvexen Verblendfläche 12 ein Spalt 14 vorliegt.

Die zur Kontaktfläche 13 gegenüberliegende Seite der Keramikschale 10 stellt die vestibuläre, anatomische Zahnform 15 dar. Der Spalt 14 zwischen der konvexen Verblendfläche 12 des Verblendgerüsts 11 und der konkaven Kontaktfläche 13 der Keramikschale 10 wird mittels einer keramischen Füllmasse die typischerweise als Keramik-Sol bereit gestellt wird, eliminiert. Dabei fungiert die viskose keramische Füllmasse als Ausgleichsmasse, und kann entsprechend unterschiedliche Spaltmaße sowie Hinterschnitte zwischen der Keramikschale 10 und dem Verblendgerüst 11 ausgleichen. Der Füllmasse, d.h. das Keramik/Glas-Sol soll hier ungleiche Spaltmaße oder Hinterschnitte des Gerüstes oder des Formenkörpers als feuerfeste Masse ausgleichen, um die dadurch hervorgerufenen Einfallstellen an der Verblendung oder der Außenfläche der anatomischen Zahnform 15 zu vermeiden. Dazu wird die Keramikschale 10 nach einem formgebenden Anpassen an das Verblendgerüst 11 mit der viskosen keramischen Füllmasse hinterspritzt und auf das Verblendgerüst 11 gedrückt, so dass die überschüssige Füllmasse herausgepresst wird.

Aufgrund der flexiblen Eigenschaft der Keramikschale 10 kann somit der Formverlauf beispielsweise an der Präparationsgrenze durch Biegen und Schneiden individuell angepasst werden. Wichtig ist jedoch, dass durch die Verwendung der viskosen keramischen Füllmasse eine unkontrollierte Formveränderung der vestibulären, anatomischen Zahnform 15 verhindert wird. Ein ungleichmäßiger Spalt 14 würde sich auf die vestibuläre anatomische Zahnform 15 übertragen, so dass die als Verblendschale genutzte Keramikschale 10 mit einer veränderten äußeren Form an dem Verblendgerüst befestigt wäre. Ebenso könnten Einfallstellen an der dentalen Verblendung entstehen.

Durch die viskose keramische Füllmasse wird eine unerwünschte thermische Formveränderung durch Aufschmelzen und Schrumpfen weitestgehend kompensiert. Dabei kann es auch vorgesehen sein, der Füllmasse UV-Initiatoren zur Lichtpolymerisation beizufügen, so dass bis zum ersten Brandschritt die Keramikschale 10 über die Füllmasse auf dem Verblendgerüst 11 fixiert wird.

In Fig. 4 ist eine zweite Ausführungsform gezeigt. Hierbei wird im Unterschied zu der oben beschriebenen Ausführungsform nach Fig. 3 die Keramikschale 10 nicht auf ein Verblendgerüst sondern auf einen feuerfesten Formkörper 16 zum Beispiel in einer Anwendung als eine vollkeramische Restauration aufgebracht. Die weitere Verfahrensführung bzw. Befestigung ist jedoch identisch.

Demnach kann die Keramikschale 10 sowohl zur Bildung von vollkeramischem Zahnersatz in Form einer Krone oder Kappe mit palatinalem Anteil als auch für die Verblendung eines Dentalgerüsts eingesetzt werden.

Nach dem ersten Brandschritt können noch weitere Brandschritte zur Farb- und Formkorrektur durchgeführt werden. Die als Folienkeramik hergestellt Keramikschale 10 ist elastisch und kann somit dem dreidimensionalen Formverlauf der Präparationsgrenze durch Biegen und Schneiden individuell angepasst werden. Es ist grundsätzlich möglich, dem Keramik-Sol einen geeigneten UV-Initiator für Lichtpolymerisation beizufügen, um die in der Form nachträglich dem Gerüst oder Träger plastisch angepasste Keramikschale 10 bis zur ersten Brandführung zu fixieren.

Die verbleibenden Freiflächen des Gerüstes können anschließend mit einer herkömmlichen Pulverkeramik-Wasser-Mischung mit einem Keramikpinsel konventionell verblendet werden, oder mit einem thixotropen Keramik-Sol in gewünschter Zahnform komplettiert werden.

Da beim ersten Hauptvakuumbrand mit einer Schrumpfung von bis zu 16% auszugehen ist, können die approximalen Bereiche und die Incisalleiste mit geeigneten Effektmassen aufgebaut werden, um zusammen mit dem Grünling im Rahmen der ersten Brandführung diesen Schrumpf zu kompensieren.

Ferner bietet die Verwendung der industriell hergestellten flexiblen Keramikschale den erhebliche Vorteil des sehr homogenes Keramikgefüges, welches zu einem gleichmäßigen Schrumpf führt. Im Gegensatz dazu weist die händische, anatomische Schichtung den maßgeblichen Nachteil auf, das hier portionsweise ein in unterschiedliche Viskosität und Feststoff/Wassergehalt angemischte Pulverkeramik Anwendung findet, welche dann zu unterschiedlich starken Schrumpfungen und Rissbildungen führt.

Die vorstehend und die in den Ansprüchen angegebenen sowie die den Abbildungen entnehmbaren Merkmale sind sowohl einzeln als auch in verschiedener Kombination vorteilhaft realisierbar. Die Erfindung ist nicht auf die beschriebenen Ausführungsbeispiele beschränkt, sondern im Rahmen fachmännischen Könnens in mancherlei Weise abwandelbar.

## Patentansprüche

1. Verfahren zur Herstellung von Zahnersatz, insbesondere mittels eines keramischen Halbzeugs (1) oder einer anatomisch geformten Keramikschale (10) als Verblendung eines Dentalgerüstes (11) oder als vollkeramischer Zahnersatz, umfassend die Schritte:
- Bereitstellen einer adaptierfähigen anatomisch geformten Keramikschale (10) mit einer konkaven Kontaktfläche (13) und einer gegenüberliegenden Außenfläche einer vestibulären, anatomischen Zahnform (15) als plastisch formbaren Grünling;
- Aufbringen der Keramikschale (10) an ein Gerüst (11) oder an einen Träger mittels einer viskosen keramischen Füllmasse als Ausgleichsmasse; wobei die Keramikschale (10) mit der viskosen keramischen Füllmasse hinterspritzt und auf das Gerüst (11) oder den Träger gesetzt wird; und
- Durchführen wenigstens eines Brennschritts unterhalb einer Temperatur von 1050°C.

2. Verfahren nach Anspruch 1, bei dem zusätzlich ein Adaptieren der anatomisch geformten Keramikschale (10) an das Gerüst (11) oder des Träger durchgeführt wird.

3. Verfahren nach Anspruch 1 oder 2, bei dem die keramische Füllmasse eine farbgebende Eigenschaft aufweist.

4. Verfahren nach Anspruch 1 bis 3, bei dem der Schritt des Bereitstellens einer anatomisch geformten Keramikschale (10) eine spanende Bearbeitung aus einem Halbzeug (1) umfasst, das als Grünling aus einer Mischung umfassend ein Keramikpulver und ein Bindersystem gefertigt ist.

5. Verfahren nach Anspruch 1 bis 3, bei dem der Schritt des Bereitstellens einer anatomisch geformten Keramikschale ein Bereitstellen eines Keramikschlickers, ein Aufbereiten des Keramikschlickers zu einem Schlickerfilm, ein eventuelles Vortrocknen des Schlickerfilms zur Anpassung des Feuchtigkeitsgrades oder der Viskosität, ein Bilden einer konkaven flexiblen Keramikschale (10) in anatomischer Form und eine Entnahme der flexiblen Keramikschale (10) aus einer Negativ-Form in Form eines Grünlings umfasst.

6. Verfahren nach Anspruch 5, bei dem zum Bilden der konkaven Keramikschale (10) ein Eindrücken des Schlickerfilms in die Negativ-Form, ein Tiefziehen oder ein Einbringen in Formplatten erfolgt.

7. Verfahren nach Anspruch 1, bei dem das Bilden der anatomisch geformten Keramikschale (10) mittels eines additiven Verfahrens, insbesondere eines 3D-Druckverfahrens oder Fräsverfahrens erfolgt.

8. Verfahren nach einem der Ansprüche 1 bis 7, bei dem der Schritt des Bereitstellens einer anatomisch geformten Keramikschale (10) mit wenigstens zwei unterschiedlich gefärbter Schichten (2, 3) erfolgt.

9. Verfahren nach einem der Ansprüche 1 bis 8, bei dem der Grünling oder ein aus dem Grünling hervorgegangener Weißling infiltriert ist.

10. Verfahren nach Anspruch 9, bei dem die Infiltration mit einer Glas- oder Keramik-Suspension vor dem Brennschritt erfolgt.

11. Verfahren nach Anspruch 9, bei dem die Infiltration mittels eines Polymers oder Harz nach dem Brennschritt erfolgt.

12. Verfahren nach einem der Ansprüche 1 bis 11, bei dem ein Brandschritt zum Dichtsintern durchgeführt wird.

13. Verfahren nach einem der Ansprüche 1 bis 12, bei dem die Keramikschale (10) als Verblendschale ausgeführt wird.

14. Verfahren nach einem der Ansprüche 1 bis 12, bei dem die Keramikschale (10) als Kappe oder Krone mit einem palatinalen Anteil ausgeführt wird.

## Claims

1. Method for producing a denture, in particular by means of a ceramic semi-finished product (1) or an anatomically shaped ceramic shell (10) as a veneer of a dental frame (11) or as a fully ceramic denture, comprising the following steps:
- providing an adaptable, anatomically shaped ceramic shell (10) having a concave contact surface (13) and an opposite outer surface of a vestibular, anatomical tooth shape (15) as a plastically moldable green body;
- applying the ceramic shell (10) to a frame (11) or to a carrier by means of a viscous ceramic filling compound as a compensating compound; wherein the ceramic shell (10) is back-injected with the viscous ceramic filling compound and is placed on the frame (11) or the carrier; and
- performing at least one firing step below a temperature of 1050°C.

2. Method according to claim 1, wherein the anatomically shaped ceramic shell (10) is additionally adapted to the frame (11) or the carrier.

3. Method according to claim 1 or claim 2, wherein the ceramic filling compound has a coloring property.

4. Method according to claim 1 to 3, wherein the step of providing an anatomically shaped ceramic shell (10) comprises machining from a semi-finished product (1) which is manufactured as a green body from a mixture comprising a ceramic powder and a binder system.

5. Method according to claim 1 to 3, wherein the step of providing an anatomically shaped ceramic shell comprises providing a ceramic slip, processing the ceramic slip to form a slip film, optionally predrying the slip film to adjust the degree of moisture or the viscosity, forming a concave, flexible ceramic shell (10) in an anatomical shape, and removing the flexible ceramic shell (10) from a negative mold in the form of a green body.

6. Method according to claim 5, wherein the slip film is pressed into the negative mold, is deep-drawn, or is inserted into mold plates to form the concave ceramic shell (10).

7. Method according to claim 1, wherein the anatomically shaped ceramic shell (10) is formed by means of an additive method, in particular a 3D printing method or milling method.

8. Method according to one of claims 1 to 7, wherein the step of providing an anatomically shaped ceramic shell (10) is carried out with at least two differently colored layers (2, 3).

9. Method according to one of claims 1 to 8, wherein the green body or a white body resulting from the green body is infiltrated.

10. Method according to claim 9, wherein the infiltration is carried out using a glass suspension or ceramic suspension before the firing step.

11. Method according to claim 9, wherein the infiltration is carried out by means of a polymer or resin after the firing step.

12. Method according to one of claims 1 to 11, wherein a baking step is performed to achieve dense sintering.

13. Method according to one of claims 1 to 12, wherein the ceramic shell (10) is designed as a veneer shell.

14. Method according to one of claims 1 to 12, wherein the ceramic shell (10) is designed as a cap or crown having a palatal portion.

## Revendications

1. Procédé de fabrication de prothèse dentaire, en particulier au moyen d'un produit semi-fini (1) céramique ou d'une coque céramique (10) de forme anatomique comme placage d'une ossature dentaire (11) ou comme prothèse dentaire entièrement céramique, comprenant les étapes :
- de fourniture d'une coque céramique (10) de forme anatomique présentant une surface de contact (13) concave I, laquelle coque peut être adaptée, et une surface extérieure opposée d'une forme de dent (15) vestibulaire et anatomique sous forme de corps cru plastiquement déformable ;
- d'application de la coque céramique (10) sur une ossature (11) ou sur un support au moyen d'une masse de remplissage céramique visqueuse comme masse d'équilibrage ; la masse de remplissage céramique visqueuse étant injectée dans la coque céramique (10), laquelle coque céramique est placée sur l'ossature (11) ou le support ; et
- de réalisation d'au moins une étape de cuisson en dessous d'une température de 1 050 °C.

2. Procédé selon la revendication 1, dans lequel la coque céramique (10) de forme anatomique est en outre adaptée à l'ossature (11) ou au support.

3. Procédé selon la revendication 1 ou 2, dans lequel la masse de remplissage céramique présente une propriété colorante.

4. Procédé selon les revendications 1 à 3, dans lequel l'étape de fourniture d'une coque céramique (10) de forme anatomique comprend l'usinage à partir d'un produit semi-fini (1) réalisé sous forme de corps cru à partir d'un mélange comprenant une poudre céramique et un système liant.

5. Procédé selon les revendications 1 à 3, dans lequel l'étape de fourniture d'une coque céramique de forme anatomique comprend la fourniture d'une barbotine céramique, la préparation de la barbotine céramique pour former un film de barbotine, éventuellement le préséchage du film de barbotine afin d'ajuster le degré d'humidité ou la viscosité, la formation d'une coque céramique (10) flexible concave de forme anatomique, et l'extraction, sous la forme d'un corps cru, de la coque céramique (10) flexible d'un moule négatif.

6. Procédé selon la revendication 5, dans lequel, pour former la coque céramique (10) concave, le film de barbotine est pressé dans le moule négatif, embouti ou inséré dans des plaques de moulage.

7. Procédé selon la revendication 1, dans lequel la formation de la coque céramique (10) de forme anatomique est effectuée au moyen d'un procédé additif, en particulier d'un procédé d'impression 3D ou d'un procédé de fraisage.

8. Procédé selon l'une des revendications 1 à 7, comprenant l'étape de fourniture d'une coque céramique (10) de forme anatomique comportant au moins deux couches (2, 3) de couleurs différentes.

9. Procédé selon l'une des revendications 1 à 8, dans lequel le corps cru ou un corps blanc dérivé du corps cru est infiltré.

10. Procédé selon la revendication 9, dans lequel l'infiltration d'une suspension de verre ou céramique est effectuée avant l'étape de cuisson.

11. Procédé selon la revendication 9, dans lequel l'infiltration avec un polymère ou une résine est effectuée après l'étape de cuisson.

12. Procédé selon l'une des revendications 1 à 11, dans lequel une étape de cuisson est réalisée pour assurer l'étanchéité du frittage.

13. Procédé selon l'une des revendications 1 à 12, dans lequel la coque céramique (10) est réalisée sous la forme d'une coque de placage.

14. Procédé selon l'une des revendications 1 à 12, dans lequel la coque céramique (10) est réalisée sous la forme d'une coiffe ou d'une couronne comportant une partie palatine.
